# EUROPÄISCHE PATENTSCHRIFT

(11) **EP 1 364 621 B1**
(45) Veröffentlichungstag und Bekanntmachung des Hinweises auf die Patenterteilung: **23.06.2004**
(21) Anmeldenummer: 03018817.1
(22) Anmeldetag: 26.02.1998
(51) Int. Cl.: A61B 17/70

(54) **Vorrichtung zum Positionieren und Fixieren von Knochen und/oder Knochenfragmenten**
Device for positioning and fixing bone and/or bone fragments
Dispositif pour la mise en place et fixation des os et/ou des fragments d'os

(30) Priorität: 26.02.1997 DE 19707677
(43) Veröffentlichungstag der Anmeldung: 26.11.2003
(62) Teilanmeldung aus: 98103359.0
(73) Patentinhaber: Deutsches Zentrum für Luft- und Raumfahrt e.V., 51147 Köln (DE)
(72) Erfinder: Balazs, Matthias, Dr., 82284 Grafrath (DE); Wolf, Oleg, Dr., 13125 Berlin (DE)
(74) Vertreter: von Kirschbaum, Albrecht, Dipl.-Ing.

(56) Entgegenhaltungen:
- WO-A-91/06254
- WO-A-96/29947
- DE-A- 19 512 709

## Beschreibung

Die Erfindung betrifft eine Vorrichtung zum Positionieren und Fixieren von Knochen und/oder Knochenfragmenten nach dem Oberbegriff des Anspruchs 1.

Eine aus WO 96/29 947 bekannte Vorrichtung weist eine Pedikelschraube, die aus einem Schraubekörper mit Außengewinde und einer Hülse mit zum Außengewinde passenden Innengewinde besteht, und ein Verbindungselement in Form einer sogenannten Kugelkopfschraube auf. Dies bedeutet zum einen, vor oder während einer Operation müssen diese Teile der Pedikelschraube montiert d.h. miteinander verschraubt werden. Ferner ist durch das Vorsehen von zwei Gewinden keine eindeutige und damit zielgerichtete Montage der Pedikelschraube, der Kugelkopfschraube und der auf die Pedikelschraube geschraubten Hülse bezüglich einer Lochplatte mit Hilfe einer Sicherungsmutter möglich.

Wird die Sicherungsmutter fest angezogen, ist nicht eindeutig, welches der beiden Gewinde dadurch festgelegt wird. So kann beispielsweise sowohl eine Verschraubung zwischen der Pedikelschraube und der Hülse als auch eine Verschraubung zwischen einem Kugelstift und der Sicherungsmutter festgelegt werden, ohne daß die jeweils andere Verschraubung ordnungsgemäß festgezogen sein muß. Somit weiß ein operierender Chirurg bei der aus WO 96/29 947 bekannten Vorrichtung nicht genau, ob das Implantat wirklich festsitzt oder ob es unter Umständen noch locker ist.

Ferner kann bei dieser bekannten Vorrichtung eine Pedikelschraube nicht mittels eines Werkzeug in einen Knochen eingeschraubt werden. Darüber hinaus kann bei der bekannten Vorrichtung beim Einschrauben einer Sicherungsmutter das Anzugsmoment nicht eindeutig abgestützt werden; das bedeutet, sowohl die Pedikelschraube als auch gegebenenfalls der Kugelkopf des Verbindungselements können verdreht werden. Dadurch kann jedoch das Implantat eine präzise vorherbestimmte Ausrichtung verlieren.

Weiterhin ist bei der bekannten Vorrichtung nachteilig, dass sich die Verschraubung in unerwünschter Weise lösen bzw. gegebenenfalls sogar festziehen kann; hierdurch wird dann entweder ein definiertes Festziehen der Schraubverbindung unmöglich oder das Verbindungselement mit Kugelkopf wird in einer unerwünschten Richtung verklemmt, so dass dadurch eine gezielte Ausrichtung verhindert ist. Obendrein besitzt die Sicherungsmutter keine zu einer Öffnung in der Lochplatte korrespondierende Profilierung, so dass eine eindeutige Ausrichtung bzw. Orientierung zwischen Sicherungsmutter und der Lochplatte nicht einwandfrei gewährleistet ist.

Aus 195 12 709 A1 ist eine weitere Vorrichtung bekannt, bei welcher Elemente der Vorrichtung wiederum in der richtigen Reihenfolge zusammensetzt werden müssen. Obendrein ist die Handhabung dieser Vorrichtung sehr umständlich, da ein frei präparierten Knochenbereich nur eingeschränkt zugänglich ist.

Aus DE 27 47 312 A ist zum Verbinden von Knochenteilen eine Schraube mit abgerundeten Flanken bekannt. Darüber hinaus ist die mechanische Festigkeit und damit die Sicherheit des Implantats durch starke Sprünge im Querschnitt eingeschränkt. Obendrein sind die Wandstärken der bekannten Vorrichtung schwach ausgelegt und starke Kraftumlenkungen erforderlich. Darüber hinaus erfordert bei der bekannten Vorrichtung die Werkstoffbelastung aufgrund von mechanischen Spannungen beachtliche Querschnitte, wodurch die Vorrichtung insgesamt erheblich voluminöser ausgeführt werden muss.

Aus WO91/06254 ist eine Pedikelschraube bekannt, die zusammen mit einer Korrektur- und Haltevorrichtung für die Wirbelsäule verwendet wird. Damit beim Aufspreizen zweier Wirbelkörper zum Aufrichten eines dazwischen liegenden Wirbelkörpers letzterer durch die Einwirkung des Weichteilmantels zur Innenseite der Krümmung der Wirbelsäule hin nicht seitlich versetzt wird, ist die Pedikelschraube so ausgebildet, dass sie einen Gewindeschaftteil mit einem kugelsegment- bzw. kugelförmigen Kopf sowie einen darauf aufsetzbaren Aufsatz mit einer zu dem Kopf passenden Lagerfläche an seinem einen Ende und ein mit dem Aufsatz verbundenes Element aufweist. Mittels dieses Elements ist eine Verbindung zu einer Stange der Korrektur- und Haltevorrichtung hergestellt. Hierbei sind das Element und der Aufsatz so miteinander verbunden, dass der Abstand zwischen dem die Lagerfläche aufweisenden einem Ende und dem Element veränderbar ist. Um jedoch die Höhenverstellung der Vorrichtung zu ermöglichen, muss der Kopf in dem Lager drehbar und schwenkbar bleiben.

Aufgabe der Erfindung ist es daher, eine Vorrichtung zum Positionieren und Fixieren von Knochen und/oder Knochenfragmenten so auszubilden, dass ein Ausrichten der Knochen und/oder Knochenfragmente zueinander in einer bestimmten Höhenlage zueinander möglich ist.

Gemäß der Erfindung ist diese Aufgabe durch die Merkmale im Anspruch 1 gelöst. Vorteilhafte Weiterbildungen sind Gegenstand der auf den Anspruch 1 unmittelbar oder mittelbar rückbezogenen Ansprüche.

Gemäß einer bevorzugten Ausführungsform der Erfindung hat jedes Befestigungselement einen zylindrischen Kern und mit einem Schraubgewinde konstanten Durchmessers, der über einen Ansatz in ein kugelförmiges Kopfteil übergeht. Ferner ist ein Zwischenteil als eine Hülse ausgebildet, welche - bezogen auf das Befestigungselement - aus einem oberen und einem unteren Hülsenteil zusammengesetzt ist. Hierbei weist das untere Hülsenteil zur Aufnahme des kugelförmigen Kopfteils des Befestigungselements einen kugelsegmentförmigen Bereich mit darunter anschließendem kegelstumpfförmigen Bereich auf. Das obere Hülsenteil weist einen durchgehenden, teilweise mit Innengewinde versehenen zylindrischen Bereich auf.

In diesen zylindrischen Bereich des oberen Hülsenteils ist das als Gewindestange ausgeführte Verbindungselement frei wählbarer Länge einschraubbar. Hierzu ist in dem unteren, dem Befestigungselement zugewandten Ende des als Gewindestange ausgeführten Verbindungselements eine der Wölbung des kugelförmigen Kopfteils entsprechende konkave Vertiefung ausgebildet. Durch diese konkave Vertiefung ist sichergestellt, dass das Befestigungselement in jeder gewünschten Stellung bezüglich eines Knochens und/oder Knochenfragmenten fixierbar ist.

Gemäß der Erfindung kann somit die Befestigungsschraube in einem einstellbaren Abstand zu der Lochplatte fixiert werden. Hierzu ist das kugelförmige Kopfteil der Befestigungsschraube in einem zu dem kugelförmigen Kopfteil komplementären Teil im Inneren der aus den zwei Hülsenteilen zusammengesetzten Hülse gelagert. Da die Hülse an dem der Befestigungsschraube abgewandten Ende eine zylindrische Aussparung mit Innengewinde aufweist, lässt sich in die Hülse das als Gewindestange ausgebildete Verbindungsteil einschrauben. Ferner ist die Hülse bzw. zumindest das obere Hülsenteil zum Ansetzen eines Werkzeugs, beispielsweise eines Maulschlüssels, außen als Sechskant ausgebildet.

Somit ist bei dieser Ausführungsform besonders vorteilhaft, dass der Abstand zwischen Lochplatte und der jeweiligen Befestigungsschraube über die Gewindestange, deren Länge ohnehin entsprechend den jeweils vorliegenden Erfordernissen wählbar ist, frei einstellbar ist. Um den Kopfteil der Befestigungsschraube zuverlässig fixieren zu können, weist die Gewindestange an dem Kopfteil der Befestigungsschraube zugewandten Ende einen der Wölbung des kugelförmigen Kopfteils entsprechende konkave Ausnehmung auf. Ferner ist der als Gewindestange ausgebildete Verbindungsteil mittels zweier Muttern beispielsweise in einem Langloch der Lochplatte höheneinstellbar und in dieser Lage fixierbar.

Nachfolgend wird die Erfindung anhand bevorzugter Ausführungsformen unter Bezugnahme auf die anliegenden Zeichnungen im einzelnen erläutert. Es zeigen:
- Fig.1a: eine Schnittansicht einer bevorzugten Ausführungsform entlang einer Linie III - III in Fig.1b;
- Fig.1b: eine Schnittansicht dieser Ausführungsform entlang einer Linie IV - IV in Fig.2a;
- Fig.1c: eine Vorderansicht einer weiteren bevorzugten Ausführungsform sowie
- Fig.2a bis 2d: bevorzugte Ausführungsformen von Lochplatten, die zum Positionieren und Fixieren von Knochen und/oder Knochenfragmenten verwendbar sind.

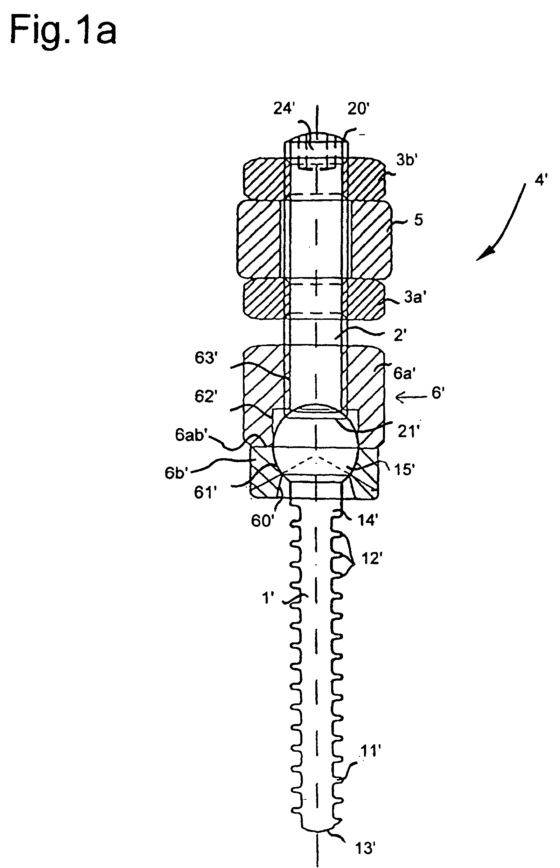

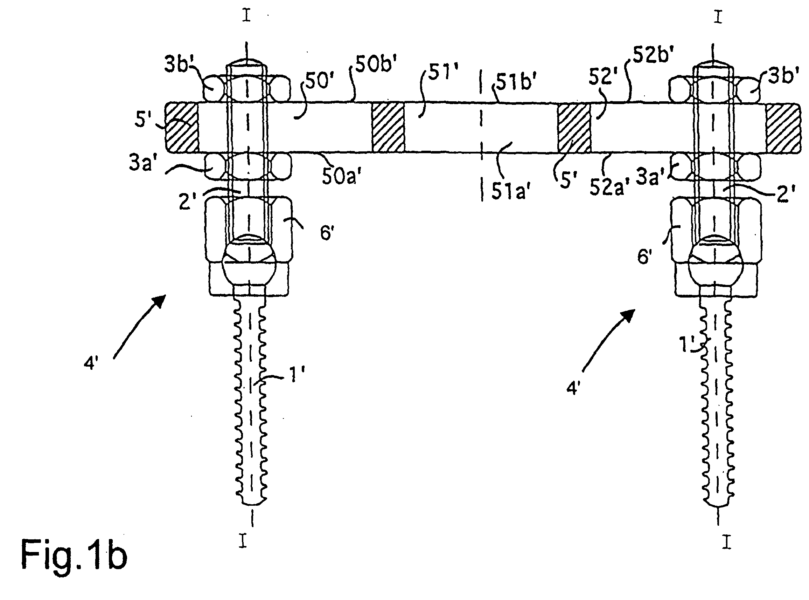

Anhand von Fig.1a bis 1c wird nachstehend eine bevorzugte Ausführungsform gemäß der Erfindung beschrieben. In der in Fig.1a wiedergegebenen Schnittansicht entlang der Linie I-I in Fig.1b sind eine Haltevorrichtung 4', die aus einer Befestigungsschraube 1', einem Zwischenteil in Form einer Hülse 6' und einem als Gewindestange ausgeführten Verbindungsteil 2' besteht, Sicherungsmuttern 3a' und 3b' sowie eine Lochplatte 5 dargestellt.

Der mit einem Schraubgewinde 11' versehene Bereich der Befestigungsschraube 1' weist einen zylindrischen Kern auf, auf dem das Schraubgewinde 11' mit konstantem Außendurchmesser vorgesehen ist. Die Flankenenden 12' des Schraubgewindes 11' sind ebenso wie das freie Ende 13' abgerundet. Am oberen Ende des Schraubgewindes 11' ist ein ringförmiger Ansatz 14' ausgebildet, dessen Außendurchmesser dem Außendurchmesser des Schraubgewindes 11' entspricht. Der ringförmige Ansatz 14' geht in einen kugelförmigen Kopfteil 15' über, der in einer Hülse 6' aufgenommen ist.

Die Hülse 6' besteht aus fertigungstechnischen Gründen aus zwei Hülsenteilen 6a' und 6b', welche im montierten Zustand fest miteinander verbunden sind. Das in Fig.1a untere Hülsenteil 6b' weist eine durchgehende Ausnehmung auf, deren in Fig.1a unteres Ende ein kegelstumpfförmiger Bereich 60' ist. An den kegelstumpfförmigen Bereich 60' grenzt ein kugelsegmentförmiger Bereich 61' an, dessen Innendurchmesser dem Außendurchmesser des kugelförmigen Kopfteils 15' der Befestigungsschraube 1' entspricht.

Das in Fig.1a obere Hülsensteil 6a', das im montierten Zustand an einer gemeinsamen Berührungsfläche 6ab' mit dem Hülsenteil 6b' verbunden ist, weist seinerseits eine durchgehende Aussparung auf, die von einem in Fig.1a unten liegenden zylindrischen Bereich 62' in einen mit einem Innengewinde versehenen zylindrischen Bereich 63' übergeht. Vorzugsweise das Hülsenteil 6a' ist als Sechskant ausgebildet, um ein entsprechendes Werkzeug, gegebenenfalls einen gekröpften Maulschlüssel ansetzen zu können.

In das Innengewinde des Hülsenteils 6a', das vorzugsweise als ein Feingewinde ausgeführt ist, ist das als eine Gewindestange ausgeführte Verbindungsteil 2' eingeschraubt, dessen Gewinde dem vorzugsweise als Feingewinde ausgeführten Innengewinde des Hülsenteils 6a' entspricht. Die Gewindestange 2' weist an ihrem dem kugelförmigen Kopfteil 15' der Befestigungsschraube 1' benachbarten Ende 21' eine konkave Auswölbung auf, deren Wölbung derjenigen des kugelförmigenn Kopfteils 15' entspricht. Das andere Ende 20' der Gewindestange 2' ist abgerundet.

In dem in Fig.1a oberen Endteil der Gewindestange 2' ist außerdem eine zur gestrichelt wiedergegebenen Mittenachse symmetrische Ausnehmung 24' vorgesehen, die vorzugsweise eine einem Imbusschlüssel oder TROX-Schlüssel entsprechende Form aufweist. Auf die Gewindestange 2' wird eine in Fig.1a untere Mutter 3a' geschraubt. Sobald die Gewindestange 2' durch ein in Fig.1a nicht näher bezeichnetes Langloch einer Lochplatte 5' gesteckt ist, wird auf das freie obere Ende der Gewindestange 2' die Mutter 3b' geschraubt, wodurch dann zumindest die Gewindestange 2' bezüglich der Lochplatte 5' fixiert ist.

In Fig.1b, in welcher die Lochplatte 5' im Schnitt wiedergegeben ist, sind zwei Befestigungsschrauben 1' über Hülsen 6', Gewindestangen 2' und jeweils zwei Muttern 3a' und 3b' mit der Lochplatte 5' verbunden. Die Lochplatte 5' weist in Fig.1b drei Langlöcher 50' bis 52' auf, deren mittlere Bereiche in Draufsicht rechtwinklig sind und deren Endbereiche in Draufsicht jeweils halbkreisförmig ausgebildet sind. Daher können die Muttern 3a' und 3b' einfache Muttern sein, die jeweils auf der der Lochplatte 5' zugewandten Seite eben ausgeführt und lediglich außen angefast sind.

Um jedoch die Gewindestangen 2' in den Langlöchern 50' bis 52' der Lochplatte 5' besser und genauer zu positionieren, können deren Kanten 50a' bis 52b' angefast sein. In diesem Fall müssen jedoch auch die Muttern 3a' und 3b' zumindest auf einer Seite die den Anfasungen der Kanten 50a' bis 52a' bzw. 50b' bis 52b' entsprechende Form aufweisen.

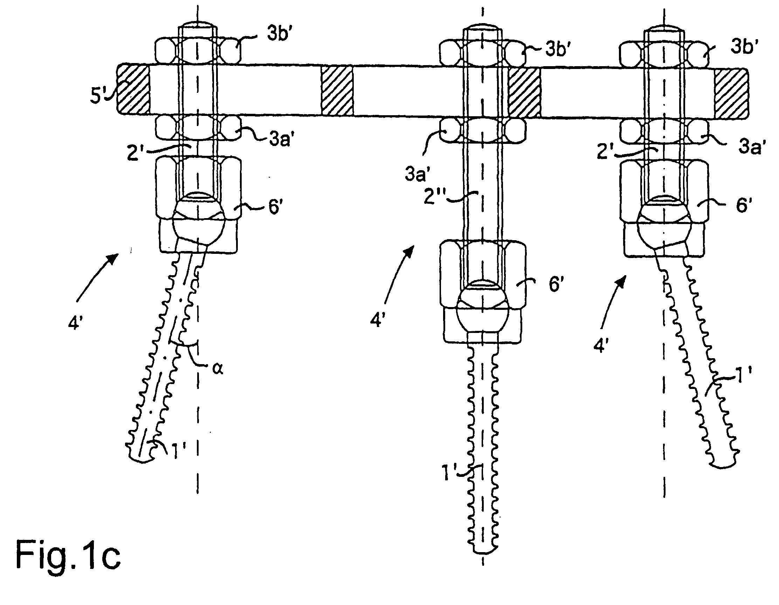

In der in Fig.1c dargestellten Vorderansicht der erfindungsgemäßen Vorrichtung schließen die strichpunktiert eingetragenen Mittenachsen der Befestigungsschrauben 1' der beiden äußeren, an der Lochplatte 5' befestigten Halterungsvorrichtungen 4' und die gestrichelt wiedergegebenen verlängerten Mittenachsen der Verbindungsteile 2' einen Winkel α ein. In Fig.1c ist eine dritte, in der Mitte wiedergegebene Halterungsvorrichtung 4' an der Lochplatte 5' fixiert, bei welcher die gestrichelte Mittenachse der Befestigungsschraube 1' und der Gewindestange 2" fluchten.

Außerdem ist Fig.1c zu entnehmen, dass bei der bevorzugten Ausführungsform der Erfindung der Länge der Gewindestangen 2' bzw. 2'' frei wählbar ist. Dadurch ist auch die Lage der jeweiligen Halterungsvorrichtung 4' bezüglich der Lochplatte 5' bzw. der Abstand der Unterseite der Hülse 6' von der Lochplatte 5' entsprechend den jeweiligen Gegebenheiten wählbar und diesen anpaßbar. Eine Feineinstellung bzw. präzise Einstellung der Position ist dann insbesondere durch die Wahl der Position der in Fig.1c unteren Schrauben 3a' gegeben.

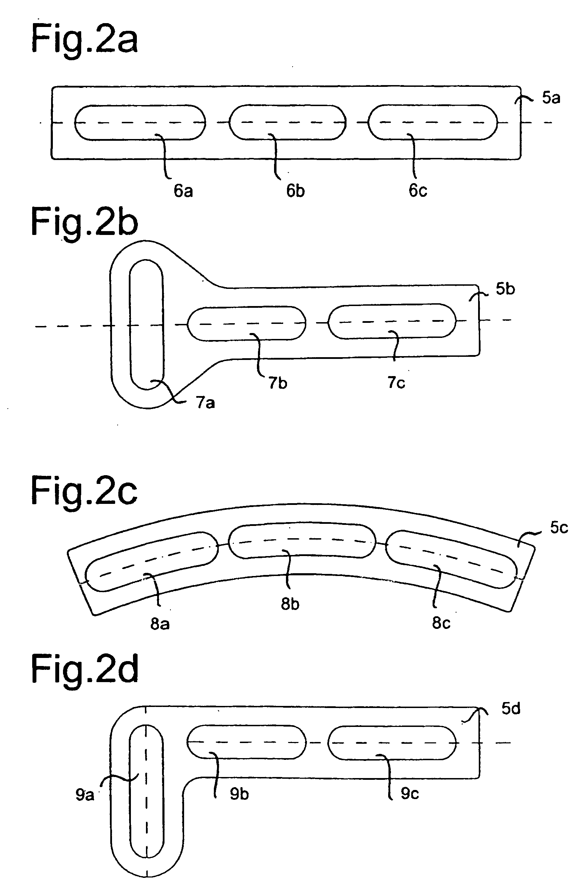

In Fig.2a bis 2d sind beispielhaft in Draufsiccht mögliche Ausführungsformen von Lochplatten 5a, 5b, 5c und 5d dargestellt. Fig.2a zeigt eine Lochplatte 5a, die drei Langlöcher 6a, 6b und 6c aufweist, die symmetrisch zu einer gestrichelt wiedergegebenen Mittellinie angeordnet sind und, wie auch in Fig.2a dargestellt, unterschiedliche Längen haben können.

In Fig.2b ist eine T-förmige Lochplatte 5b dargestellt, die zwei in Reihe angeordnete, in Fig.2b gleich große Langlöcher 7b und 7c und ein zu diesen beispielsweise senkrecht angeordnetes Langloch 7a aufweist.

In Fig.2c ist eine bogenförmige Lochplatte 5c dargestellt, die beispielsweise drei gleichgroße Langlöcher 8a, 8b und 8c aufweist, die entlang einer gestrichelten gekrümmten Linie ausgerichtet sind.

In Fig.2d ist eine L-förmige Lochplatte 5d dargestellt, die zwei in Reihe hintereinander angeordnete, in Fig.3d gleich große Langlöcher 9b, 9c sowie ein zu diesen senkrechtes Langloch 9a aufweist.

Ferner sind auch andere beliebige Kombinationen von unterschiedlich bemessenen Langlöchern denkbar. Die in Fig.2a bis 2d dargestellten Lochplatten sind als in sich eben dargestellt. Die einzelnen Lochplatten können auch so vorgeformt sein, dass beispielsweise ihre beiden Endbereiche nach oben abgewinkelt sind oder ein Endbereich nach unten und der andere nach oben abgewinkelt ist oder überhaupt nur ein Endbereich abgewinkelt bzw. gekröpft ist, während der andere Endbereich eben ausgeführt ist.

### Bezugszeichenliste

- 1': Befestigungsschraube
- 11': Schraubgewinde
- 12': Flankenenden von 1'
- 13': freies Ende von 1'
- 14': ringförmiger Ansatz
- 15': kegelförmiges Kopfteil

- 2': Verbindungsteil/Gewindestange
- 20', 21': Enden
- 24': symmetrische Ausnehmung

- 3a', 3b': Muttern
- 4': Haltevorrichtung
- 5': Lochplatte
- 50' bis 52': Langlöcher
- 50a' bis 52b': Kanten
- 5a bis 5d: Lochplatten

- 6': Hülse
- 6a', 6b': Hülsenteile
- 60': kegelstumpfförmiger Bereich
- 61': kugelsegmentförmiger Bereich
- 62': zylindrischer Bereich

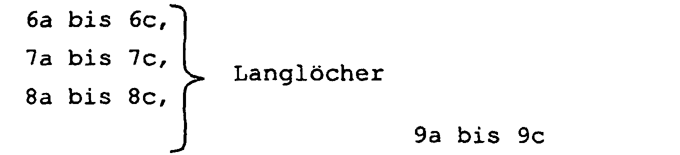

## Patentansprüche

1. Vorrichtung zum Positionieren und Fixieren von Knochen und/oder Knochenfragmenten, mit einer als Prothesenplatte dienenden Lochplatte (5') und Haltevorrichtungen (4'), bestehend jeweils aus einem Befestigungselement (1') mit Schraubgewinde (11'), einem Zwischenteil und einem Verbindungselement mit Außengewinde, und mit Sicherungsmitteln (3a', 3b'), so dass die Haltevorrichtungen (4) mittels der Sicherungsmittel (3) an der Lochplatte (5) befestigbar und fixierbar sind, wobei
jedes Befestigungselement (1') einen zylindrischen Kern mit Schraubgewinde (11') konstanten Durchmessers hat, der über einen Ansatz (14') in ein kugelförmiges Kopfteil (15') übergeht; **dadurch gekennzeichnet, dass**
das Zwischenteil als Hülse (6') ausgebildet ist, welche - bezogen auf das Befestigungselement (1') - aus einem oberen und einem unteren Hülsenteil (6a', 6b') besteht, wobei
das untere Hülsenteil (6b') zur Aufnahme des kugelförmigen Kopfteils (15') des Befestigungselements (1') einen kugelsegmentförmigen Bereich (61') mit darunter anschließendem kegelstumpfförmigen Bereich (60') aufweist und
das obere Hülsenteil (6a') einen durchgehenden teilweise mit Innengewinde versehenen zylindrischen Bereich (63') aufweist, in welchen
das als Gewindestange ausgeführte Verbindungselement (2') frei wählbarer Länge, in dessen unteren, dem Befestigungselement (1') zugewandten Ende eine der Wölbung des kugelförmigen Kopfteils (15') entsprechende, konkave Vertiefung ausgebildet ist, einschraubbar ist, so dass das Befestigungselement (1') in jeder gewünschten Stellung fixierbar ist.

2. Vorrichtung nach Anspruch 1, **dadurch gekennzeichnet, dass** zumindest das obere Hülsenteil (6a') der Hülse (6') eine als Sechskant ausgebildete Außenfläche hat.

3. Vorrichtung nach Anspruch 1 und 2, **dadurch gekennzeichnet, dass** am freien oberen Ende des als Gewindestange ausgeführten Verbindungselements (2') ein zu dessen Mittenachse symmetrische Ausnehmung (24') vorgesehen ist.

## Claims

1. A device for positioning and fixating bones and/or bone fragments comprising a slotted plate (5') serving as prosthetic plate and anchoring devices (4') each consisting of a fastener element (1') including a screw thread (11'), an intermediate member and a connecting element with male thread and locking means (3a', 3b'), so that said anchoring devices (4') can be secured and fixated to said slotted plate (5) by means of locking means, whereby
each fastener element (1') comprises a cylindrical core with a screw thread (11') of constant diameter, which translates into a spherical head part (15') through a lug (14'), **characterized in that**
the intermediate member is designed as sleeve (6') which - related to the fastener element (1') - consists of an upper and a lower sleeve part (6a', 6b'), whereby
the lower sleeve part (6b') comprises for accommodating the spherical head part (15') of the fastener element (1') a portion (61') in the shape of a spherical segment with a portion (60') in the form of a truncated cone adjoining there below, and the upper sleeve part (6a') comprises a continuous portion (63') partially provided with a female thread, in which
the connecting element (2') designed as thread rod of a freely selectable length can be screwed into, in the lower end of which facing the fastener element (1') a corresponding, concave recess is configured, so that the fastener element (1') can be fixated at any position desired.

2. The device as set forth in claim 1, **characterized in that** at least the upper sleeve part (6a') of the sleeve (6') has an outer surface configured as hexagonal.

3. The device as set forth in claims 1 and 2, **characterized in that** on the free upper end of the connecting element (2') designed as thread rod there is provided a recess (24') symmetrical to the central axis thereof.

## Revendications

1. Dispositif pour le positionnement et la fixation d'os et/ou de fragments d'os, comportant une plaque perforée (5') utilisée en tant que plaque de prothèse et des dispositifs de retenue (4'), constitués chacun par un élément de fixation (1') pourvu d'un filetage de vis (11'), une partie intermédiaire et un élément de liaison pourvu d'un filetage extérieur, et des moyens de sécurité (3b, 3b') de sorte que les dispositifs de retenue (4') peuvent être fixés et bloqués à l'aide des moyens de fixation sur la plaque perforée (5'), dans lequel chaque élément de fixation (1') possède un noyau cylindrique comportant un filetage de vis (11') de diamètre constant, qui se prolonge par l'intermédiaire d'un embout (14') par une partie de tête sphérique (15');
**caractérisé en ce que**
la partie intermédiaire est agencée sous la forme d'une douille (6'), qui - d'une manière rapportée à l'élément de fixation (1') - est constituée par une partie supérieure et une partie inférieure de douille (6a', 6b'),
la partie inférieure (6b') de la douille comportant, pour loger la partie de tête de forme sphérique (15') de l'élément de fixation (1'), une partie en forme de segment sphérique (61') comportant une partie de forme tronconique (60') qui se raccorde au-dessous de la partie précédente, et la partie supérieure (6a') de la douille possédant une partie cylindrique continue (63'), qui est pourvue partiellement d'un taraudage, dans lequel
l'élément de liaison (2') réalisé sous la forme d'une tige filetée possédant une longueur pouvant être choisie librement, dans l'extrémité, tournée vers l'élément de fixation (1'), duquel est réalisé un renfoncement concave, qui correspond au cintrage de la partie de tête de forme sphérique (15'), peut être vissé, de sorte que l'élément de fixation (1') peut être fixé dans n'importe quelle position désirée.

2. Dispositif selon la revendication 1, **caractérisé en ce qu'**au moins la partie supérieure (6a') de douille (6') possède une surface extérieure agencée sous la forme d'un profil hexagonal.

3. Dispositif selon les revendications 1 et 2, **caractérisé en ce que** sur l'autre extrémité de l'élément de liaison (2') agencé sous la forme d'une tige filetée est prévu un évidement (24') qui est symétrique par rapport à son axe médian.
